# FASCICULE DE BREVET EUROPEEN

(11) **EP 0 649 656 B1**
(45) Date de publication et mention de la délivrance du brevet: **16.08.2001**
(21) Numéro de dépôt: 94402384.5
(22) Date de dépôt: 24.10.1994
(51) Int. Cl.: A61K 31/473

(54) **Compositions pharmaceutiques à base d'acriflavine utiles pour le traitement de l'herpès et du zona**
Acriflavine enthaltende Arzneimittel verwendbar zur Behandlung von Herpes und Zona
Pharmaceutical compositions based on acriflavine useful for the treatment of herpes and zona

(30) Priorité: 22.10.1993 FR 9312618
(43) Date de publication de la demande: 26.04.1995
(73) Titulaire: LABORATOIRE M. RICHARD, 26740 Sauzet (FR)
(72) Inventeur: Richard, Marcel, F-07200 Aubenas (FR); Froissant-Richard, Solange, F-26740 Saint Marcel-Les Sauzet (FR)
(74) Mandataire: Gillard, Marie-Louise

(56) Documents cités:
- WO-A-90/14086
- DE-A- 4 137 548
- CHEMICAL ABSTRACTS, vol. 83, no. 1, 7 Juillet 1975, Columbus, Ohio, US; abstract no. 1407p, 'DIFFERENT SUSCEPTIBILITY TO ACRIFLAVINE IN TWO STRAINS OF HERPESVIRUS HOMINIS WITH DIFFERENT CYTOPATHIC EFFECT' & REVISTA LATINOAMERICANA DE MICROBIOLOGIA, vol.16, no.4, 1974 pages 217 - 225
- ANTIMICROBIAL AGENTS AND CHEMOTHERAPY, vol.10, no.2, 1976 pages 234 - 240 W. ROHDE ET AL. 'HYDROXYQUINOLINES INHIBIT RIBONUCLEIC ACID-DEPENDENT DEOXYRIBONUCLEIC ACID POLYMERASE AND INACTIVATE ROUS SARCOMA AND HERPES SIMPLEX VIRUS'
- CUTIS, vol.19, no.1, 1977 pages 98 - 100 F.W. ROSENBERG ET AL. 'WIDESPREAD CUTANEOUS HERPES SIMPLEX TYPE II INFECTION'
- MED. ASPECTS HUM. SEX., vol.12, no.12, 1978 pages 103 - 104 SCHNEIDMAN H. 'DERMATOLOGIC MANIFESTATIONS OF VENERAL DISEASE'
- 'THE MERCK INDEX' 1989 , MERCK & CO., INC. , RAHWAY, N.J., U.S.A. * page 20 * * page 769 - page 770 *

## Description

La présente invention concerne le domaine médical. Plus particulièrement, l'invention concerne l'utilisation combinée d'acriflavine et d'un composé hydroxy-8 quinoléine pour la préparation d'une composition pharmaceutique utile pour le traitement de l'herpès et du zona.

L'acriflavine, qui appartient à la famille des acridines, est un mélange de chlorure de 3,6-diamino-10-méthylacridinium avec de la 3,6 acridine diamine, représenté par la formule suivante :

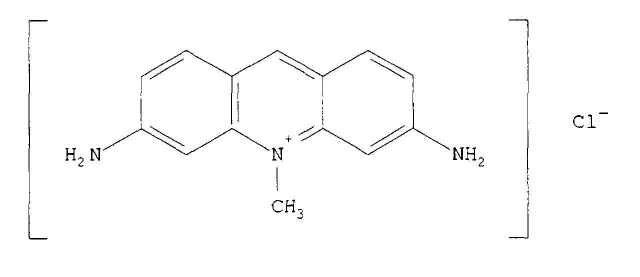

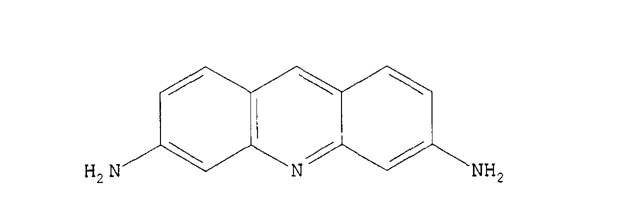

Ce produit se présente sous la forme d'une poudre rouge orangé, soluble dans l'eau, partiellement soluble dans l'alcool et pratiquement insoluble dans l'éther ou le chloroforme. Il est connu pour ses propriétés antiseptiques (voir "The Merck Index", 10ème édition, abrégé N°120).

Ce produit peut également être utilisé pour le traitement de certains troubles dermatologiques, notamment pour le traitement de l'herpès. On peut citer à ce sujet les articles parus dans "Revista latinoamericana de Microbiologia (1974), 16(4), 217-225", "Cutis (1977), 19(1), 98-100" ou bien encore "Med. Aspects Hum. Sex (1978), 12(12), 103-104".

La demande de brevet DE-A-4 137 548 décrit une combinaison à base d'acriflavine, d'acide lactique et de chlorhexidine, qui possède une activité contre le virus de l'herpès simplex.

La Demanderesse a maintenant trouvé que l'utilisation combinée d'acriflavine et d'un composé hydroxy-8 quinoléine permet de préparer des compositions pharmaceutiques utiles pour le traitement de l'herpès et du zona.

L'objet de l'invention concerne donc l'utilisation combinée d'acriflavine, ou d'un de ses sels pharmaceutiquement acceptables, et d'un composé hydroxy-8 quinoléine pour la préparation de compositions pharmaceutiques à usage humain et vétérinaire, destinées au traitement de l'herpès et du zona. En particulier, ces compositions pharmaceutiques sont utiles pour le traitement des infections vaginales et buccales de type herpétique.

Parmi les composés hydroxy-8 quinoléine que l'on peut utiliser dans le cadre de l'invention, on peut citer notamment l'hydroxy-8 quinoléine, le benzoyloxy-8 quinoléine ou le sulfate d'hydroxy-8 quinoléine (voir "The Merck Index", 10ème édition, abrégés Nos. 4765, 1115 et 4766). Avantageusement, on utilise le sulfate d'hydroxy-8 quinoléine. Ce produit se présente sous la forme d'une poudre jaune pâle, soluble dans l'eau, faiblement soluble dans l'alcool et insoluble dans l'éther.

Généralement, l'acriflavine est combinée avec le composé hydroxy-8 quinoléine de telle manière que le rapport pondéral acriflavine/composé hydroxy-8 quinoléine soit compris entre 0,75 et 5. Avantageusement, on utilise la combinaison acriflavine/composé hydroxy-8 quinoléine dans un rapport pondéral égal à 0,75, à 1 ou à 5. Cette combinaison, dans le rapport pondéral indiqué ci-dessus, permet d'obtenir une forte potentialisation de l'acriflavine pour son action inhibitrice sur la multiplication des virus de l'herpès et du zona.

Un autre objet de l'invention concerne donc la potentialisation de l'acriflavine par un composé hydroxy-8 quinoléine tel que défini précédemment.

Les sels pharmaceutiquement acceptables de l'acriflavine comprennent le chlorhydrate, le bromhydrate, le sulfate, l'hydrogénosulfate, le méthanesulfonate, le méthylsulfate, le maléate, le fumarate et le 2-naphtalènesulfonate.

Selon la présente invention, la combinaison acriflavine/composé hydroxy-8 quinoléine peut être utilisée telle quelle ou mélangée avec un ou plusieurs véhicules pharmaceutiquement acceptables selon les techniques classiques bien connues de l'homme du métier.

A titre d'exemple de véhicule pharmaceutiquement acceptable, on peut citer notamment le talc, la gomme arabique, le lactose, l'amidon, le stéarate de magnésium, les véhicules aqueux ou non, les dérivés paraffiniques, les glycols, par exemple le propylèneglycol, les agents mouillants, dispersants ou émulsifiants, et les conservateurs, couramment utilisés dans ce domaine.

Dans les compositions pharmaceutiques de la présente invention pour l'administration orale, parentérale ou topique, la combinaison d'acriflavine, ou d'un de ses sels pharmaceutiquement acceptables, et d'un composé hydroxy-8 quinoléine peut être administrée sous forme unitaire d'administration, en mélange avec des véhicules pharmaceutiques classiques, pour le traitement des maladies ci-dessus.

Les formes unitaires d'administration appropriées comprennent les formes orales, telles que comprimés, gélules, poudres, granules, à libération immédiate ou retardée, et les solutions ou suspensions orales ; les formes parentérales, telles que suspensions aqueuses, solutions salines isotoniques ou solutions stériles et injectables qui contiennent des agents de dispersion et/ou des mouillants pharmacologiquement compatibles; et les formes topiques, telles que crèmes, pommades ou lotions.

La quantité de principe actif à administrer par jour dépend de la particularité de l'indication thérapeutique, de la gravité des affections à traiter, ainsi que du poids du patient et de la voie d'administration.

Pour l'administration orale, on utilisera généralement entre 5 et 250 mg par jour d'acriflavine, la quantité de composé hydroxy-8 quinoléine combiné à l'acriflavine étant telle que le rapport pondéral acriflavine/composé hydroxy-8 quinoléine est compris entre 0,75 et 5.

Pour l'administration parentérale, la dose d'acriflavine peut généralement varier entre 0,5 et 25 mg par jour, celle du composé hydroxy-8 quinoléine étant telle que le rapport pondéral indiqué ci-dessus est vérifié.

Pour l'administration topique, des solutions aqueuses ou hydro-alcooliques d'acriflavine combinée à un composé hydroxy-8 quinoléine (dans le rapport pondéral indiqué ci-dessus) peuvent être appliquées 2 à 3 fois par jour.

La présente invention va maintenant être décrite plus en détail à l'aide des essais ci-après ; dans ces essais, on a utilisé les trois préparations pharmaceutiques ci-après, sous la forme de solutions aqueuses, dénommées PREPARATION 1, PREPARATION 2 et PREPARATION 3, contenant les quantités d'acriflavine, de composé hydroxy-8 quinoléine (sulfate d'hydroxy-8 quinoléine) et d'excipients suivantes (% en poids), diluées dans 100 ml d'eau :

| | **Préparation 1** | **Préparation 2** | **Préparation 3** |
|---|---|---|---|
| acriflavine | 0,15 | 0,25 | 0,20 |
| sulfate d'hydroxy-8 quinoléine | 0,20 | 0,05 | 0,20 |
| propylène glycol | 15,00 | 9,00 | 10,00 |
| alcool à 95° | 5,00 | 0,70 | 10,00 |

### Essai 1 : toxicité

La toxicité de la PREPARATION 1 est de 51 et 71 ml/kg par voie orale, déterminée sur la souris mâle et femelle de la souche SWISS.

La toxicité de la PREPARATION 2 est de 51 et 50,7 ml/kg par voie orale, 10,1 et 11 ml/kg par voie intraveineuse, déterminée sur la souris mâle et femelle de la souche SWISS.

La toxicité des PREPARATIONS 1 et 2 est supérieure à 50 ml/kg par voie orale et de 10 ml/kg par voie intraveineuse, déterminée sur le rat mâle et femelle de la souche WISTAR.

### Essai 2 : activité antiherpétique

### 1/ Essai in vitro

- les PREPARATIONS 1 et 2 présentent une activité virucide sur le virus de l'herpès simplex type I (HSV I) puisqu'elles entraînent un abaissement en titre viral de 4 log dans l'expérimentation réalisée selon la norme AFNOR activité virucide T 72.180.
- l'activité virucide et anti-virale de la PREPARATION 3 sur une souche du virus HSV I a également été testée et on a comparé cette activité à celle de l'acriflavine seule et à celle du sulfate d'hydroxy-8 quinoléine seul; ces tests ont été effectués à des doses infra-cytotoxiques. Dans un premier temps, on a donc déterminé l'activité virucide des différents composés testés par mise en contact du virus avec ces composés. Puis on a déterminé l'activité anti-virale des composés testés en inoculant le virus (sous la forme d'une suspension préalablement diluée dans du milieu de Hanks de manière à avoir une concentration en virus de 100 DE₅₀ pour 10 µl de suspension) à une culture de fibroblastes de poumon embryonnaire (cellules commercialisées par la société Bio-Mérieux sous la référence MCR5), puis en traitant la culture cellulaire, inoculée par le virus, par les composés à tester, dilués également dans du milieu de Hanks. Les plaques de cultures cellulaires ont ensuite été observées au microscope inversé après 24h et 48h d'inoculation, ainsi que des cultures témoins non-inoculées et des virus témoins incubés sans composés. Les résultats sont rassemblés dans le tableau suivant:

| Composés | Concentrations minimales (µg/ml) activité virucide activité anti-virale | |
|---|---|---|
| ACF 0,2 %* | 1 | 2 |
| 8-HQS 0,2 % * | -** | -** |
| PREPARATION 3 | 0,65 | 1 |

| | | |
|---|---|---|
| * excipients identiques à ceux de la préparation 3 | | |
| * * absence d'activité | | |

ACF : acriflavine
8-HQS : sulfate d'hydroxy-8 quinoléine

On peut tout d'abord noter à la lecture de ce tableau que le sulfate d'hydroxy-8 quinoléine ne présente pas de propriétés virucide et anti-virale intrinsèques. On constate ensuite que la combinaison acriflavine 0,2% / sulfate d'hydroxy-8 quinoléine 0,2% (Préparation 3) présente une activité virucide et anti-virale à des doses minimales deux fois inférieures à celles de l'acriflavine seule, ce qui est surprenant compte tenu de l'inactivité du sulfate d'hydroxy-8 quinoléine. Il y a donc potentialisation de l'acriflavine par le sulfate d'hydroxy-8 quinoléine, ce qui est intéressant au niveau thérapeutique.

### 2/ Essai in vivo

L'expérimentation a été réalisée en application locale sur des cobayes albinos (Dunkin-Hartley, IFFA-CREDO) auxquels on a préalablement inoculé par scarification le virus Herpès simplex type I. Sur ces scarifications, on a appliqué 0,1 ml d'une solution de virus HSV I titrant 10 000 DI₅₀ pour 100 *µ*l. Un flanc des animaux a servi de témoin. Le traitement a été effectué deux fois par jour pendant 3 jours, la première application ayant lieu 18 h après l'inoculation.

Les paramètres retenus pour déterminer la gravité des lésions cutanées sont : érythèmes, indurations, nombre et taille des vésicules, aires d'infection et présence ou absence de virus. Les observations sont réalisées 5, 6, 7 et 8 jours après l'inoculation.

On a testé sur ce modèle les PREPARATIONS 1, 2 et 3, ainsi que l'acriflavine seule, le sulfate d'hydroxy-8 quinoléine seul et l'acyclovir (2-amino-1,9-dihydro-9-[(2-hydroxyethoxy)methyl]-6H-purin-6-one), qui est un agent antiviral spécifique.

Les PREPARATIONS 1, 2 et 3 et l'acriflavine présentent des propriétés antivirales sur le virus HSV I; ces produits réduisent significativement l'infection herpétique cutanée. Les érythèmes, les indurations, le nombre et la sévérité des vésicules et le pourcentage de réponses positives sont significativement améliorés. Le sulfate d'hydroxy-8 quinoléine n'a aucun effet sur les lésions cutanées. L'activité anti-virale des composés testés, en pourcentage moyen d'activité constatée entre le jour 5 et le jour 8, est indiquée dans le tableau ci-après :

| Composés | concentration (%) | % moyen d'activité |
|---|---|---|
| ACF* | 0,2 | 0,0 |
| | 1,0 | 44,5 |
| | 2,0 | 81,5 |
| | 4,0 | 95,2 |
| 8-HQS * | 0,2 | 0,0 |
| Préparation 3 | 0,2/0,2 | 54,7 |
| Acyclovir pommade | 3,0 | 97,7 |
| Acyclovir** | 1,0 | 55,2 |

| | | |
|---|---|---|
| * excipients identiques à ceux de la préparation 3 | | |
| ** solution dans le polyéthylèneglycol | | |

On peut s'apercevoir à la lecture de ce tableau que la préparation 3 (combinaison acriflavine 0,2% / sulfate d'hydroxy-8 quinoléine 0,2%) a une activité anti-virale comparable à celle de l'acyclovir à 1%. Il est également intéressant de noter que la préparation 3 présente une activité anti-virale comparable à celle de l'acriflavine seule, mais à des doses en acriflavine environ 6 à 7 fois inférieures. La potentialisation de l'acriflavine par le sulfate d'hydroxy-8 quinoléine est donc très importante et bien plus marquée que dans les essais *in vitro*. Cet effet est d'autant plus surprenant que le sulfate d'hydroxy-8 quinoléine n'a aucune activité anti-virale. La combinaison acriflavine/sulfate d'hydroxy-8 quinoléine selon l'invention est donc très avantageuse sur le plan pharmacologique, notamment en ce qui concerne la tolérance, puisqu'elle fait intervenir de faibles doses d'acriflavine.

A titre d'exemple, on a préparé les compositions pharmaceutiques suivantes :

| **Constituants** | **composition 1** | **composition 2** |
|---|---|---|
| Acriflavine | 0,150 g | 0,250 g |
| Sulfate d'hydroxy-8 quinoléine | 0,200 g | 0,050 g |
| Propylène glycol | 15 g | 9,0 g |
| Alcool à 95° | 5 g | 0,7 g |
| Chlorure de magnésium | 0,750 g | 0 |
| Paraoxybenzoate de méthyle | 0,100 g | 0,100 g |
| Eau purifiée qsp | 100 ml | 100 ml |

Comme exemple d'application thérapeutique, l'acriflavine a donné des résultats favorables en clinique humaine :
- dans les infections herpétiques buccales et vulvaires
- dans les zonas puisqu'il réduit significativement les éruptions vésiculeuses et les névralgies des territoires cutanés infectés.

## Revendications

1. Utilisation combinée de l'acriflavine, ou d'un de ses sels pharmaceutiquement acceptables, et d'un composé hydroxy-8 quinoléine, pour la préparation d'une composition pharmaceutique pour le traitement de l'herpès et du zona.

2. Utilisation selon la revendication 1, caractérisée en ce que le composé hydroxy-8 quinoléine est l'hydroxy-8 quinoléine, le benzoyloxy-8 quinoléine ou le sulfate d'hydroxy-8 quinoléine.

3. Utilisation selon la revendication 2, caractérisée en ce que le composé hydroxy-8 quinoléine est le sulfate d'hydroxy-8 quinoléine.

4. Utilisation selon l'une quelconque des revendications 1 à 3, caractérisée en ce que le rapport pondéral acriflavine/composé hydroxy-8 quinoléine est compris entre 0,75 et 5.

5. Utilisation selon la revendication 4, caractérisée en ce que le rapport pondéral acriflavine/composé hydroxy-8 quinoléine est égal à 0,75.

6. Utilisation selon la revendication 4, caractérisée en ce que le rapport pondéral acriflavine/composé hydroxy-8 quinoléine est égal à 1.

7. Utilisation selon la revendication 4, caractérisée en ce que le rapport pondéral acriflavine/composé hydroxy-8 quinoléine est égal à 5.

8. Utilisation d'un composé hydroxy-8 quinoléine choisi parmi l'hydroxy-8 quinoléine, le benzoyloxy-8 quinoléine ou le sulfate d'hydroxy-8 quinoléine comme agent potentialisateur de l'activité de l'acriflavine pour inhiber la multiplication des virus de l'herpès et du zona.

## Claims

1. Combined use of acriflavine or of one of its pharmaceutically acceptable salts, and an 8-hydroxyquinoline compound for the manufacture of a pharmaceutical composition for the treatment of herpes and zona.

2. Use according to claim 1, characterized in that the 8-hydroxyquinoline compound is 8-hydroxyquinoline, 8-benzoyloxyquinoline or 8-hydroxyquinoline sulfate.

3. Use according to claim 2, characterized in that the 8-hydroxyquinoline compound is 8-hydroxyquinoline sulfate.

4. Use according to any one of claims 1 to 3, characterized in that the weight ratio acriflavine/8-hydroxyquinoline compound is between 0.75 and 5.

5. Use according to claim 4, characterized in that the weight ratio acriflavine/8-hydroxyquinoline compound is equal to 0.75.

6. Use according to claim 4, characterized in that the weight ratio acriflavine/8-hydroxyquinoline compound is equal to 1.

7. Use according to claim 4, characterized in that the weight ratio acriflavine/8-hydroxyquinoline compound is equal to 5.

8. Use of an 8-hydroxyquinoline compound selected from 8-hydroxyquinoline, 8-benzoyloxyquinoline and 8-hydroxyquinoline sulfate as an agent potentiating the activity of acriflavine to inhibit the multiplication of the herpes and zona viruses.

## Patentansprüche

1. Kombinierte Verwendung von Acriflavin oder einem seiner pharmazeutisch annehmbaren Salze und einer 8-Hydroxychinolin-Verbindung zur Herstellung einer pharmazeutischen Zusammensetzung für die Behandlung von Herpes und Zoster.

2. Verwendung nach Anspruch 1, dadurch gekennzeichnet, dass die 8-Hydroxychinolin-Verbindung 8-Hydroxychinolin, 8-Benzoyloxychinolin oder 8-Hydroxychinolinsulfat ist.

3. Verwendung nach Anspruch 2, dadurch gekennzeichnet, dass die 8-Hydroxychinolin-Verbindung 8-Hydroxychinolinsulfat ist.

4. Verwendung nach einem der Ansprüche 1 bis 3, dadurch gekennzeichnet, dass das Gewichtsverhältnis Acriflavin/8-Hydroxychinolin-Verbindung zwischen 0,75 und 5 beträgt.

5. Verwendung nach Anspruch 4, dadurch gekennzeichnet, dass das Gewichtsverhältnis Acriflavin/8-Hydroxychinolin-Verbindung 0,75 beträgt.

6. Verwendung nach Anspruch 4, dadurch gekennzeichnet, dass das Gewichtsverhältnis Acriflavin/8-Hydroxychinolin-Verbindung 1 beträgt.

7. Verwendung nach Anspruch 4, dadurch gekennzeichnet, dass das Gewichtsverhältnis Acriflavin/8-Hydroxychinolin-Verbindung 5 beträgt.

8. Verwendung einer 8-Hydroxychinolin-Verbindung ausgewählt aus 8-Hydroxychinolin, 8-Benzoyloxychinolin oder 8-Hydroxychinolinsulfat als Mittel zur Potenzierung der Aktivität von Acriflavin zur Inhibition der Vermehrung von Herpes- und Zosterviren.
